# EUROPEAN PATENT APPLICATION

(11) **EP 4 591 851 A1**
(43) Date of publication of application: **30.07.2025**
(21) Application number: 22958964.3
(22) Date of filing: 22.09.2022
(51) Int. Cl.: A61K 8/92, A61Q 5/00, A61K 8/9783

(54) **PREBIOTIC COSMETIC COMPOSITIONS, USE OF THE PREBIOTIC COSMETIC COMPOSITIONS, USE OF BABASSU OIL, AND COSMETIC METHOD**

(71) Applicant: Natura Cosméticos S.A., 05106-000 São Paulo SP (BR)
(72) Inventor: DE MIRANDA CHAVES VASQUEZ PINTO, Luciana, 05106-000 São Paulo - SP (BR); PANZARIN SAVIETTO, Joice, 05106-000 São Paulo - SP (BR); BRUSSOLO BIDOIA, Guilherme, 05106-000 São Paulo - SP (BR)
(74) Representative: Simmons & Simmons
(86) International application number: PCT/BR2022/050374
(87) International publication number: WO 2024/059917

(57) **Abstract**

The present invention relates to prebiotic cosmetic compositions comprising babassu oil (*Orbignya oleifera*) having prebiotic activity, as well as the use of said oil, favoring the skin, hair and scalp balance, nourishing the beneficial microorganisms residing in the microbiota of these regions, protecting them from environmental aggression, strengthening them, reducing sensitivity and irritations and improving their defense systems.

## Description

### FIELD OF THE INVENTION

The present invention relates to prebiotic cosmetic compositions comprising babassu oil (*Orbignya oleifera*)*,* as well as the use of said oil having prebiotic activity, promoting the skin balance, nourishing the beneficial microorganisms residing in the skin's microbiota, protecting it from environmental aggression, strengthening it, reducing sensitivity and irritation and improving its defense system.

### PRIOR ART

Microbiota is the set of microorganisms present in a given ecosystem, such as the skin or hair.

The skin microbiota is mostly made up of bacteria, but it also contains viruses, fungi and protozoa. The microorganisms present in the skin microbiota are determined by environmental conditions such as anatomical location, moisture, sebum and sweat production, oxygen concentration, pH, among others, in addition to biological factors such as hormonal fluctuations and the individual's age.

The hair microbiota or hair flora, in turn, is a community of microorganisms that develop on the scalp and hair strands (shaft). It is composed of bacteria and fungi, which feed on sebum and keratinocytes (dead skin cells).

The microbiota is responsible for several physiological functions, such as the production of nutrients or defense peptides, modulation of the immune response and, in the case of the hair microbiota, it also acts by protecting and maintaining healthy hair.

Microorganisms can attach to the skin by binding to structures in the skin or hair, such as carbohydrates or cell receptors within skin cells or organized into colonies bound together by a biofilm.

The biofilm forms a microenvironment having pH, oxygen concentration, nutrients and metabolites different from those of the skin. It further facilitates the communication of bacteria by *quorum sensing* and protects the microbiota of endogenous and exogenous antimicrobial agents from the individual's immune system.

Human skin has approximately 1,000 bacteria for every cell. Genetic sequencing studies show that bacteria exist not only on the skin surface, but also in sweat and sebaceous glands, follicles, skin invaginations and even in the deeper layers of the skin such as the dermis.

The most common genera present in the superficial layers of the skin are *Staphylococcus, Cutibacterium, Micrococcus* and *Corynebacteria.*

These bacteria are distributed across different regions of the skin depending on the microenvironment. *Staphylococcus* and *Corynebacteria,* for example, are the most abundant genera in the skin and remain throughout life. *Cutibacterium,* which are anaerobic, are present in the sebaceous glands. *Corynebacteria* are present in the armpits and are related to the production of malodor. Fungi of the genus *Malassezia* are present on the scalp and are related to seborrheic dermatitis and dandruff.

Human skin, in general, provides the microbiota with a dry, slightly acidic environment with only proteins and lipids available.

Microbiota balance involves adhesion mechanisms, interaction with the individual's immune defense system, changes in the microenvironment, nutrient consumption and production of antimicrobial agents.

The superficial layers of the skin are constantly being peeled off, which removes part of the microbiota. The ability to adhere to viable skin cells ensures that the microorganisms remain on the skin. Bacteria bind to membrane receptors on cells, such as those of the *toll-like* family that recognize patterns associated with bacteria (TLR 1-10), mannose receptors, or NOD-like receptors. These receptors trigger immune pathways that discriminate between pathogenic and beneficial bacteria, stimulating appropriate responses. The microbiota composition teaches the immune system to discriminate beneficial bacteria more accurately, modulating inflammatory responses and reducing the skin's sensitivity to external factors such as variations in temperature, humidity and exposure to UV radiation.

Furthermore, the microbiota produces lactic acid which, together with free fatty acids (from sebum), acidic amino acids (from sweat) and urocanic and pyrrolidone carboxylic acids (from the keratinization process of corneocytes) maintain the skin's pH more acidic, restricting the microorganisms present in the microbiota. Lipids are the main source of carbon used by the skin microbiota.

Variations in age, sex and hormonal cycle interfere with the nutrients availability for the microbiota, which can lead to fluctuations in the microorganism composition.

The role of the selective barrier against the external environment performed by the skin, is greatly facilitated by antimicrobial peptides (AMPs) produced both by keratinocytes and by the skin microbiota. More than 2,000 molecules having antimicrobial activity produced by microorganisms and catalogued in public and private databases have been described so far. Among them are α- and β-defensins (hBD), cathelicidins, C-type lectins, dermicidins, etc. Most AMPs are composed of small peptides having about 2 to 15kDa. The imbalance (excess or lack) of AMPs in the skin is related to pathologies such as atopic dermatitis, rosacea and psoriasis. Some factors produced by the skin, such as vitamin D and PTH, regulate the synthesis of AMPs. AMPs are important for protecting the skin against pathogens, controlling the microbiota population and limiting contact between keratinocytes and the microbiota. AMPs activity is generally broad spectrum, affecting both Gram-positive and Gram-negative bacteria and, in some instances, fungi, protozoa and viruses.

The microbiota also produces other substances, such as hyaluronic acid produced by *Streptococcus* and some *Lactobacillus,* which is a constituent of the extracellular matrix that fills the dermis, giving shape to the skin and stimulating the production of β-defensin 2 (hBD2).

Sphingomyelinase is produced by keratinocytes, *Streptococcus* and *Lactobacillus,* being an enzyme that participates in the synthesis of ceramides that are essential for maintaining the skin barrier.

Lipoteichoic acid (LTA) is the structural component of Gram-positive bacteria such as *Staphylococcus, Lactobacillus* and *Bifidobacteria,* which stimulates the production of antimicrobial peptides such as β-defensins (hBD) and cathelicidins.

Peptidoglycans are structural components of the cell wall of Gram-positive bacteria, which modulate the individual's immune response by stimulating the production of IL-8 by keratinocytes and recruiting phagocytes to infected sites.

Lactic acid, an alpha hydroxy acid produced by *Lactobacillus* and *Bifidobacteria,* stimulates the skin peeling process by weakening cell adhesions, stimulating the production of ceramides by keratinocytes, strengthening the skin barrier and composing the NMF, which retains skin moisture.

Acetic acid, which is produced by *Lactobacillus* and *Bifidobacteria,* has antibacterial activity against S. *aureus* and *P. aeruginosa.*

The main representatives of the skin microbiota include *Staphylococcus epidermidis,* the major component of the skin, which are halotolerant and capable of growing in environments with low water activity. It is generally harmless to the skin, but can cause nosocomial infections by colonizing foreign bodies such as catheters and implants in predisposed and immunocompromised individuals. These are extremely resistant to AMPs, do not attack keratinocytes and produce AMPs (epidermin, Pep5, epilancin K7 and epicidin 280) that control the *S*. *aureus* and *Streptococcus* populations.

However, removal of *S*. *epidermidis* from the skin, for example, by the excessive use of topical antibiotics, leaves the individual immunocompromised and very susceptible to infections by pathogens.

In addition, they synthesize proteases, keratinases, lipases and nucleases that participate in the physiological skin turnover. They synthesize phenol-soluble modulins (PSMs) that are proteins having surfactant and selective antibacterial properties for *S*. *aureus* and *Streptococcus* by dissolving the biofilm formed by such pathogenic bacteria.

*Staphylococcus aureus* can be found in healthy skin. It is the main pathogen in human skin and is responsible for folliculitis, boils, subcutaneous abscesses, meningitis, endocarditis and septicemia. Conditions such as atopic dermatitis, viruses (Herpes simplex type I or Papillomavirus) and opportunistic fungi (*Trichophyton rubrum*) predispose the skin to S. *aureus* infections.

The increasing number of strains resistant to antibiotics such as metacycline and vancomycin, make *S*. *aureus* a serious problem in hospital settings. It synthesizes bacteriocins such as staphylococcin 462 that inhibits other *S*. *aureus* strains.

*Corynebacteria jeikeium* and *Corynebacteria striatum* are found in healthy skin, being the most present microorganisms following *S*. *epidermidis.* They can be found in the axillary, inguinal and perineal regions due to their high tolerance to salt concentrations. They adhere to keratinocytes at an estimated ratio of 25 bacteria/cell through fibronectin receptors. They produce bacteriocins such as lacticidin Q, which is responsible for the synthesis of dermicidin and cathelicidin present in sweat, proposing a skin defense activity.

*Cutibacterium acnes* present in healthy microbiota in regions such as the face and the back is a saprophytic, anaerobic microorganism found mainly in the sebaceous glands and being associated with manifestations such as folliculitis and acne vulgaris. The main source of energy used by it are lipids and fatty acids present in sebum. The presence of porphyrin leaves *C*. *acnes* quite sensitive to UV radiation and stimulates the production of IL-8 and IL-12 by keratinocytes, forming a pro-inflammatory microenvironment.

Studies show *C*. *acnes* as being involved in the process of hypercorneification of the pilosebaceous duct, hence exacerbating inflammatory processes. In addition, it synthesizes acetic acid and propionic acid as well as lipases and proteases. It further synthesizes several bacteriocins such as propionicin PLG-1, jensenin G, propionicin SM1, SM2, T1 and acnein, having activity against several lactic bacteria, Gram negative bacteria, fungi and molds.

*Malassezia furfur* It is a fastidious fungus present in the healthy microbiota in regions such as the scalp and the back, being mainly responsible for opportunistic infections such as seborrheic dermatitis, dandruff and tinea versicolor (or pityriasis). Its main source of carbon is free fatty acids. It further synthesizes an enzyme capable of degrading the microorganism cell wall, contributing to the protection of the skin against pathogens.

An imbalance in the microbiota can lead to seborrheic dermatitis, acne vulgaris, atopic dermatitis, among other diseases.

The skin's microbiota is constantly removed by the skin's natural process of desquamation and renewal. Thus, the ability of MOs to adhere to viable skin cells, including in the deepest layers such as the dermis, is a competitive advantage.

The presence of microbiota modulates the skin's inflammatory response by reducing sensitivity to factors such as temperature fluctuations, humidity and exposure to UV radiation.

The microbiota produces lactic acid, which helps to maintain the skin's pH more acidic, antimicrobial peptides (AMPs) that protect the skin from pathogens and whose imbalance is related to pathologies such as dermatitis, rosacea and psoriasis, help to regulate the production of vitamin D and are involved in the production of extracellular matrix proteins such as hyaluronic acid and enzymes important for maintaining the skin barrier such as sphingomyelinase.

*Staphylococcus epidermidis* is the main microorganism in the skin microbiota and remains so, even during aging. *S. epidermidis* is harmless to keratinocytes and produces AMPs (epidermin, Pep5, epilancin K7, soluble phenolic modulins -PSMs- and epicidin 280) that control *S*. *aureus* and *Streptococcus* populations, hence protecting the skin.

*S*. *epidermidis* synthesize proteases, keratinases, lipases and nucleases that contribute to the physiological skin turnover and play a role in maintaining skin health, protecting it from harmful opportunistic microorganisms and favoring physiological mechanisms of skin renewal.

In the scalp are hair follicles having openings that contribute to a significant increase in the surface area capable of being colonized by microbiota. The follicle is filled with sebum, debris and microorganisms. Regarding the bacteria population present on the scalp, bacteria population of the genus *Cutibacterium* spp. (mainly *Cutibacterium acne*) and the genus *Stapylococcus* spp. are the most abundant ones. Concerning the fungal population, the genus *Malassezia* spp. stands out as the most abundant one. Disruption of the scalp microbiota can lead to dysbiosis in the region, favoring local inflammation of the scalp skin.

In the instance of hair, microbiota is often disrupted by changes in hair pH (caused by Brazilian Blowout, hair straightening, coloring, etc.), antibiotic treatments, pollution, as well as the use of products containing ingredients such as sulfates.

There are numerous active ingredients available that are suggested to directly or indirectly act on the microbiota, through topical or oral administration, for example, acetic acid, diacetyl, lactic acid, hyaluronic acid, among others. And, despite the microbiota components and their direct and indirect effects on the skin being known, the common use of antibacterial agents, which affect both the beneficial and harmful bacteria present on the skin, still persists. Its indiscriminate use weakens the skin's natural defenses and leaves it more vulnerable to harmful bacteria.

For this reason, new prebiotic and probiotic ingredients are still being researched.

Prebiotics are ingredients that support beneficial bacteria. Probiotics are microorganisms that interact with the skin's natural microbiota and stimulate it to produce its own defenses. The probiotic can also act as a selective antibacterial, producing substances that control the proliferation of harmful bacteria.

Numerous state-of-the-art publications indicate that prebiotic agents can selectively favor beneficial bacteria and, consequently, are candidates for composing cosmetic compositions for this purpose. However, despite the fact that the state of the art has screened and suggested possible prebiotic ingredients, it is limited in terms of providing effective prebiotic ingredients with proven *in vitro* efficacy and with an elucidated mechanism of action, particularly for topical administration and, for this reason, it requires further investigation.

Patent document BR112018073293-1 by Natura, discloses prebiotic cosmetic compositions that may comprise babassu starch. Starches in general are good for adsorbing skin oils, however, when considering their application in cosmetic formulations, they do not dissolve, they are only dispersed and in this aspect oils can be incorporated more easily into the formulas, thus expanding the possibilities of use in different galenic formulas. Ingredients in oily form are also good emollients and film formers.

Thus, there is still a need for cosmetic compositions, particularly for topical application, that have a proven prebiotic effect on the skin.

### BRIEF DESCRIPTION OF THE FIGURES

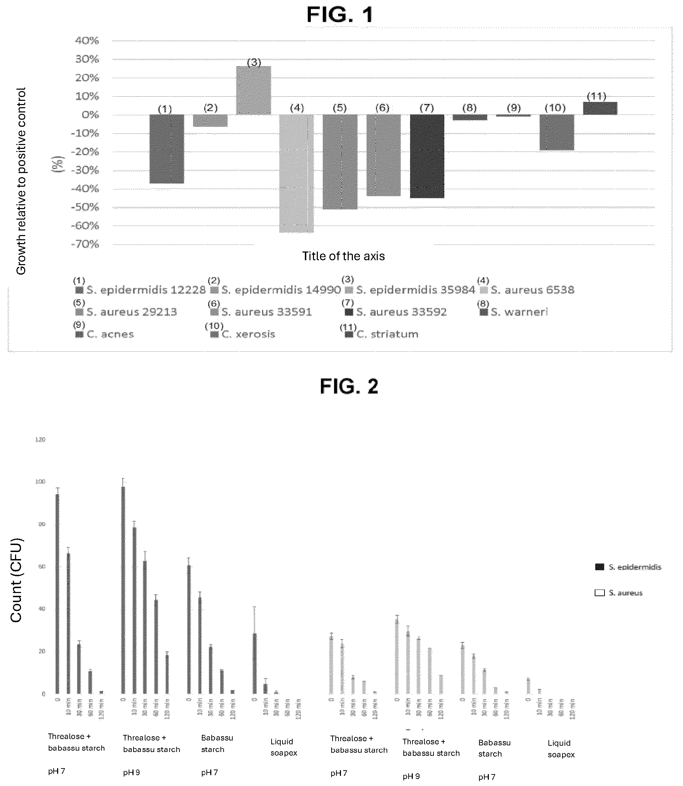

Figure 1 shows growth relative to the positive control.
Figure 2 shows the result on the proliferation of *S*. *epidermis* and *S*. *aureus* with different ingredients.

### DESCRIPTION OF THE INVENTION

In a first aspect, the present invention relates to prebiotic cosmetic compositions comprising babassu oil *(Orbignya oleifera)* having prebiotic activity, promoting the skin balance, nourishing the beneficial microorganisms that live in the skin's microbiota, protecting it from environmental aggression, strengthening it, reducing sensitivity and irritation and improving its defense system, having proven effectiveness *in vitro.*

In particular, babassu oil is used at a concentration of 0.5 to 1%, particularly around 0.01%, relative to the total weight of the composition.

According to the present invention, babassu oil is obtained from palm trees in the Maranhão region, being supplied by the COPPALJ community (*Cooperativa de Pequenos Produtores Agroextrativistas do Lado do Junco e Lago dos Rodrigues*) and has the following characteristics: it is rich in lauric, myristic, palmitic and oleic acid.

The prebiotic cosmetic compositions according to the present invention can be made available in different cosmetic forms, including without any limitation, emulsions, gels, powders, sticks, among others, including cosmetically appropriate carriers for the chosen cosmetic form.

The prebiotic cosmetic compositions according to the present invention are intended for topical application and promote the proliferation of *S*. *epidermidis,* without increasing harmful microorganisms.

Surprisingly, the prebiotic effect provided by the topical prebiotic cosmetic compositions according to the present invention is found to be efficient not only in reducing harmful bacteria on the skin, hair or scalp, favoring the beneficial bacteria in these regions, balancing their microbiota, but it also provides balance, nutrition, stimulates natural defense/protection, providing advantageous cosmetic effects, such as providing gentle formulas that do not harm the skin and scalp microbiota.

Due to these and other observed effects, the topical cosmetic compositions according to the present invention are used as cleansing and/or moisturizing products having formulas that are friendly to the skin microbiota.

The topical cosmetic compositions according to the present invention

| Ingredient | Ex. 1 | Ex. 2 | Ex. 3 | Ex. 4 |
|---|---|---|---|---|
| | (%) | (%) | (%) | (%) |
| Water | 1.2 | 2.4 | 1.2 | 2.4 |
| Ultramarines | 0.086 | 0.086 | 0.086 | 0.086 |
| Citric acid | 0.05 | 0.05 | 0.05 | 0.05 |
| Glyceryl oleate/Coco-glucoside | 2 | 2 | 2 | 2 |
| Denatonium benzoate | 0.01 | 0.01 | 0.008 | 0.008 |
| Etidronic acid | 0.1 | 0.1 | 0.038 | 0.038 |
| Perfume | 1.5 | 1.5 | 0 | 0 |
| Hydroxypropyl guar | 0.5 | 0.5 | 0.4614 | 0.4614 |
| Sodium palmitate. Sodium oleate. Sodium laurate. Sodium linoleate. Sodium stearate. Sodium myristate. Sodium caprylate. Sodium caprate. Sodium arachidate | 90.904 | 88.904 | 90.7586 | 88.7618 |
| *Orbignya oleifera seed oil* | 0.5 | 0.5 | 0.5 | 0.5 |
| Sodium Chloride | 0.35 | 0.35 | 0.35 | 0.35 |
| Titanium dioxide | 1 | 1 | 1 | 1 |
| Trehalose | 0 | 0.8 | 0 | 0.8 |
| *Zea mays* starch | 1.8 | 1.8 | 1.8 | 1.8 |

exhibit prebiotic activity for *Staphylococcus epidermidis* ATCC 35984. They did not show any prebiotic activity for non-beneficial microorganisms: *Corynebacterium xerosis* ATCC 373, *Staphylococcus aureus* ATCC 6538, *Staphylococcus aureus* ATCC 29213, *Staphylococcus aureus* ATCC 33591 e *Staphylococcus aureus* ATCC 33592. The biofilm formation test showed weak biofilm formation for all microorganisms tested, which helps prevent the adhesion and proliferation of species that are not considered beneficial on the skin.

The topical prebiotic cosmetic compositions according to the present invention may be made available in any cosmetic forms suitable for application to the face, body, hair or scalp known to the person skilled in the art for treatment or cleaning, without any limitation, including emulsions, solutions, powders, gels, pastes, soaps, shampoos, conditioners, among others.

Cosmetically acceptable excipients may be selected from compounds known in the prior art. Without limitation, they may include emollients, antioxidants, humectants, emulsifiers, surfactants, sensory or viscosity modifiers, preservatives, chelating agents, stabilizers, lubricants, thickeners, dispersants, solubilizers and other cosmetically acceptable carriers.

In another aspect, the present invention also contemplates the use of the cosmetic compositions according to the present invention, as well as babassu oil, particularly at a concentration of 0.5 to 1%, relative to the total weight of a cosmetic composition, more particularly at a concentration of 0.01%, in the nutrition of beneficial microorganisms resident in the skin, hair and scalp microbiota, protecting them from environmental aggression, strengthening them, reducing sensitivity and irritation and improving their defense systems.

The following examples, without imposing any limitation, illustrate the present invention.

### EXAMPLES

Bar soap compositions were prepared:
Liquid soap compositions were prepared:

| Ingredient | Ex. 5 | Ex. 6 | Ex. 7 | Ex. 8 |
|---|---|---|---|---|
| | (%) | (%) | (%) | (%) |
| Acrylates/C₁₀₋₃₀ alkyl acrylate crosspolymer | 0.6 | 0.6 | 0 | 0 |
| Water | 63.684 | 64.184 | 63.684 | 64.184 |
| Capryloyl/Caproyl Methyl Glucamide. Lauroyl /myristoyl methyl glucamide. Water. | 2.5 | 2.5 | 2.41 | 2.41 |

| | | | | |
|---|---|---|---|---|
| Citric acid | 0.01 | 0.01 | 0.01 | 0.01 |
| Decyl glucoside | 4.5 | 4.5 | 4.5 | 4.5 |
| Perfume | 0.5 | 0.5 | 0 | 0 |
| Glycerin | 5 | 5 | 5 | 5 |
| Hydroxyacetophenone | 0.5 | 0.5 | 0 | 0 |
| Hydroxypropyl guar | 0.1 | 0.1 | 0.0923 | 0.0923 |
| *Orbignya oleifera seed oil* | 0.005 | 0.005 | 0.005 | 0.005 |
| Potassium cocoyl glycinate. Potassium cocoate. Water | 9.5 | 9.5 | 8.8816 | 8.8816 |
| Sodium cocoamphoacetate | 10 | 10 | 9.5275 | 9.5275 |
| Sodium Gluconate | 0.1 | 0.1 | 0.1 | 0.1 |
| Sodium hydroxide | 0.001 | 0.001 | 0.001 | 0.001 |
| Trehalose | 0.5 | 0 | 0.5 | 0 |
| Propanediol | 2.5 | 2.5 | 2.5 | 2.5 |

Application of the compositions of the present invention has been shown to provide greater smoothness to the formulation and not impact the skin microbiota when compared to the same soap formulation with no babassu oil.

These results together show that the prebiotic effect provided by babassu oil when used at a specific concentration in the topical prebiotic cosmetic compositions according to the present invention is not only efficient in reducing harmful bacteria on the skin, favoring the beneficial bacteria on the skin, hair or scalp and balancing its microbiota, but also provides balance, nutrition, natural defense/protection, providing advantageous cosmetic effects, such as providing gentle formulas that do not harm the skin and scalp microbiota.

The person skilled in the art will be able to readily assess through the teachings of the instant text and examples the advantages of the invention and to propose variations and equivalent alternatives of implementation without departing from the scope of the invention as defined in the appended claims.

## Claims

1. PREBIOTIC COSMETIC COMPOSITIONS **characterized in that** they comprise babassu oil *(Orbignya oleifera)* and cosmetically acceptable excipients.

2. COMPOSITIONS, according to claim 1, **characterized in that** the babassu oil concentration ranges from 0.5 and 1% relative to the total weight of the composition.

3. COMPOSITIONS, according to claim 2, **characterized in that** the of babassu oil concentration is 0.01% relative to the total weight of the composition.

4. COMPOSITIONS, according to any one of claims 1 to 3, **characterized in that** they are for topical application to the skin, hair and scalp.

5. COMPOSITIONS, according to any one of claims 1 to 4, **characterized in that** they act in the maintenance of beneficial organisms, not favoring non-beneficial microorganisms and providing a weak biofilm for undesirable microorganisms.

6. COMPOSITIONS, according to any one of claims 1 to 4, **characterized in that** they exhibit prebiotic activity for *Staphylococcus epidermidis* ATCC 35984.

7. USE OF THE PREBIOTIC COSMETIC COMPOSITIONS, according to any one of claims 1 to 6, **characterized in that** it is in the nutrition of beneficial microorganisms resident in the skin, hair and scalp microbiota, protecting them from environmental aggression, strengthening them, reducing sensitivity and irritations and improving their defense systems.

8. USE OF BABASSU OIL **characterized in that** it is in the preparation of a cosmetic composition for the nutrition of beneficial microorganisms residing in the skin, hair and scalp microbiota, protecting them from environmental aggression, strengthening them, reducing sensitivity and irritation and improving their defense systems.

9. USE, according to claim 8, **characterized in that** the cosmetic composition comprises from 0.5 to 1% of babassu oil relative to the total weight of a cosmetic composition.

10. USE, according to claim 9, **characterized in that** the cosmetic composition comprises 0.01% babassu oil relative to the total weight of a cosmetic composition.

11. A COSMETIC METHOD **characterized in that** it comprises applying prebiotic cosmetic compositions including babassu oil *(Orbignya oleifera)* and cosmetically acceptable excipients to the skin, hair and scalp.
